(19) **Europäisches Patentamt**
**European Patent Office**
**Office européen des brevets**

(11) Veröffentlichungsnummer: **0 181 562**
**B1**

(12) **EUROPÄISCHE PATENTSCHRIFT**

(45) Veröffentlichungstag der Patentschrift: 01.08.90

(21) Anmeldenummer: 85113777.8

(22) Anmeldetag: 29.10.85

(51) Int. Cl.⁵: **C 12 N 9/14,** C 12 N 1/20, A 23 L 3/34, A 61 K 37/54, C 12 N 15/00 // (C12N1/20, C12R1:465)

(54) Bakterienlysierendes Enzymprodukt aus Streptomyceten, Verfahren zu seiner Herstellung und dafür geeigneter Stamm.

(30) Priorität: 08.11.84 DE 3440735

(43) Veröffentlichungstag der Anmeldung:
21.05.86 Patentblatt 86/21

(45) Bekanntmachung des Hinweises auf die Patenterteilung:
01.08.90 Patentblatt 90/31

(84) Benannte Vertragsstaaten:
AT BE CH DE FR GB IT LI LU NL SE

(56) Entgegenhaltungen:
EP-A-0 025 898
DE-A-2 011 935
DE-A-2 040 440

CHEMICAL ABSTRACTS, Band 93, Nr. 13, September 1980, Seite 513, Zusammenfassung Nr. 130592a, Columbus, Ohio, US; & JP-A-80 68 291 (INSTITUTE FOR PRODUCTION AND DEVELOPMENT SCIENCE) 22-05-1980

(73) Patentinhaber: HOECHST AKTIENGESELLSCHAFT
Postfach 80 03 20
D-6230 Frankfurt am Main 80 (DE)

(72) Erfinder: Wöhner, Gerhard, Dr.
Flörsheimer Strasse 27
D-6093 Flörsheim am Main (DE)
Erfinder: Voelskow, Hartmut, Dr.
Akazienstrasse 22
D-6234 Hattersheim am Main (DE)
Erfinder: Präve, Paul, Prof. Dr.
Kronberger Weg 7
D-6231 Sulzbach (Taunus) (DE)
Erfinder: Lück, Erich, Dr.
Robert-Stolz-Strasse 102
D-6232 Bad Soden am Taunus (DE)
Erfinder: VON Rymon Lipinski, Gert-Wolfhard, Dr.
Hunsrückstrasse 40
D-6230 Frankfurt am Main 80 (DE)

**Beschreibung**

Die Gewinnung eines bakterienlysierenden Enzymproduktes aus Streptomyceten ist bekannt und beispielsweise in den Deutschen Offenlegungsschriften 20 11 935, 20 40 4410 und 21 46 597 beschrieben. Die bekannten Verfahren sind — vor allem im Hinblick auf die Ausbeute an bakterienlysierenden Enzymprodukts — recht aufwendig.

Die Bildung eines bakterienlysierenden Enzymprodukts von Streptomyceten der Art coelicolor ist bisher nicht beschrieben. Eigene Untersuchungen, beispielsweise am Typ-Stamm DSM 40 233, waren ergebnislos.

Überraschenderweise wurde nun gefunden, daß von einem Stamm der Art Streptomyces coelicolor ein sehr wirksames bakterienlysierendes Enzymprodukt in hohen Ausbeuten produziert wird. Dieser Stamm wurde bei der DSM — Deutsche Sammlung von Mikroorganismen — unter der Nummer DSM 3030 hinterlegt. Dieser Stamm, seine Mutanten und Varianten, sofern sie ein bakterienlysierndes Enzymprodukt bilden, sind ein Gegenstand der Erfindung. Weitere Aspekte der Erfindung und bevorzugte Ausgestaltungen sind im folgenden wiedergegeben bzw. in den Patentansprüchen niedergelegt.

Der erfindungsgemäße Stamm wurde aus Erdproben isoliert. Selektionsmerkmal war die Exkretion eines Enzymprodukts, das in der Lage ist, bei pH 3 bis 7 Bakterien zu lysieren.

Der erfindungsgemäße Stamm wächst in üblichen Kulturmedien, in die er das bakterienlysierendes Enzymprodukt abgibt. Nach Abtrennung der Zellen verbleibt das bakterienlysierende Enzymprodukt im Kulturüberstand, aus dem es durch gängige Methoden der Proteinanreicherung und -reinigung wie Alkoholfällung, Ionenaustauscherchromatographie, Ultrafiltration und Gelfiltration isoliert werden kann.

Ein Vorteil der erfindungsgemäßen Herstellung des bakterienlysierenden Enzymprodukts ist darin zu sehen, daß in einfach zusammengesetzten Kulturmedien hohe Ausbeuten an bakterienlysierendem Enzymprodukt erzielbar sind. Besonders bewährt hat sich ein Zusatz von Zuckerrübenmelasse in einer Menge von 5 bis 50 g, vorzugsweise 10 bis 20 g pro Liter Kulturmedium.

Eine weitere Ausbeutesteigerung wird erreicht, wenn man dem Kulturmedium Calciumionen in Form leicht löslicher, ungiftiger Calciumsalze zusetzt, vorzugsweise in Form des billigen Calciumchlorids. Vorteilhaft ist eine 0,05 bis 1 molare Calciumionenkonzentration, besonders bevorzugt sind Konzentrationen von 100 bis 500 mmol/l, bespielsweise in Form eines Zusatzes von 0,2 bis 0,5 Gew.-% an Calciumchlorid-Dihydrat.

Das erfindungsgemäß erhaltene bakterienlysierende Enzymprodukt ist in einem pH-Bereich von unter 3 bis über 9 stabil und wirksam. Nach 16 Stunden Inkubation bei Raumtemperatur in Puffergemischen war die maximale Aktivität im Bereich von pH 3 bis 9 noch zu mindesten 80% erhalten.

Das Temperatur-Optimum für die Aktivität des bakterienlysierenden Enzymprodukts liegt im Bereich von 50 bis 60°C. Über diesen Bereich werden mindestens 90% der maximalen Aktivität erreicht. Von niedrigen Temperaturen ausgehend steigt die Aktivität mit steigender Temperatur bis 60°C langsam an: Bei Zimmertemperatur sind es etwa 10%, bei 30°C etwa 30%, bei 40°C etwa 60% der maximalen Aktivität. Bei höheren Temperaturen fällt die Aktivität rasch ab: Bei 65°C sind es noch etwa 30%, bei 70°C 10% der maximale Aktivität.

Das pH-Optimum für die Wirkung des bakterienlysierenden Enzymprodukts liegt bei 4,5 bis 5; 80% der Aktivität liegen im pH-Bereich von etwa 4,25 bis 5,5. Bei pH 4 sind es noch etwa 65%, bei pH 3,5 25%, bei pH 5,5 etwa 75% und bei pH 6 etwa 40% der maximalen Wirksamkeit.

Das erfindungsgemäße bakterienlysierende Enzymprodukt wirkt sehr gut auf gram-positiv und gramnegativ Bakterien. Es kann deshalb (wie handelsübliche Lysozyme) bei einer Konservierung von Lebensmitteln, zur Verhinderung von Infektionen und zur Protoplastenherstellung verwendet werden.

In den folgenden Beispielen wird die Erfindung näher erläutert. Prozentangaben beziehen sich auf das Gewicht, sofern keine anderen Angaben gemacht werden.

## Beispiel 1: Kultivierung von S. coelicolor

Schrägboden-Agarmedium, welches 2% Sojamehl, 2% Mannit und 1,5% Agar (pH 7,5) enthielt, wurde mit Streptomyces coelicolor DSM 3030 beimpft und 10 Tage bei 30°C kultiviert. Die Schrägkultur wurde mit 10 ml einer sterilen Lösung von 0,9% NaCl und 0,01% eines nichtionischen Tensids versetzt und die Sporen abgeschwemmt. 0,2 ml der Sporensuspension wurden als Impfmaterial für Schuttelkulturen von 100 ml Kulturmedium in 300 ml fassenden Kulturkolben verwendet.

Die in Tabelle 1 aufgeführten Kulturmedien Nr. 1—3 wurden angesetzt, beimpft und bei 30°C und einer Frequenz von 160 Upm geschüttelt. Nach 2, 4 und 6 Tagen wurden Proben entnommen, die Zellen abzentrifugiert und die Aktivität des bakterienlysierenden Enzymprodukts im Kulturüberstand bestimmt (Tabelle 2).

Die höchsten Ausbeuten an bakterienlysierendem Enzymprodukt wurden mit der Nährlösung 3 erhalten, die neben Glucose, Sojamehl und Caseinpepton 2% Zuckerrübenmelasse enthielt.

# EP 0 181 562 B1

## Tabelle 1: Kulturmedien

| % | Medium Nr. 1 | 2 | 3 |
|---|---|---|---|
| Glucose | 2 | | 1 |
| Mannit | | 2 | |
| Sojamehl | 2 | 1 | 0,5 |
| Caseinpepton | | | 0,5 |
| Melasse | | 1 | 2 |

## Tabelle 2: Aktivität an bakterienlysierendem Enzymprodukt im Kulturüberstand (U/ml)

| Fermentationszeit Tage | Medium Nr. 1 | 2 | 3 |
|---|---|---|---|
| 2 | 21 | 15 | 363 |
| 4 | 367 | 378 | 1106 |
| 6 | 236 | 558 | 2088 |

Beispiel 2: Ausbeuteerhöhung durch Calciumzusatz

Streptomyces coelicolor DSM 3030 wurde in der in Beispiel 1 beschriebenen Weise auf einen Nährboden, der 1% Glucose, 2% Melasse, 0,5% Sojamehl, 0,5% Caseinpepton und 0,5% $CaCl_2 \times 2H_2O$ entheilt, kultiviert. Durch den Zusatz von $CaCl_2$ wurde eine wesentliche Erhöhung der Ausbeute an bakterienlysierendem Enzymprodukt im Kulturüberstand nach 2, 4 und 6 Tagen von 768 U/ml, 2462 U/ml und 4715 U/ml erhalten.

Beispiel 3: Fermentation

10 ml einer Sporensuspension von Streptomyces coelicolor DSM 3030 nach Beispiel 1 wurden als Impfmaterial für eine Fermentation mit 5 l Kulturmedium in einem 8 l Fermenter verwendet.

Fermentationsbedingungen:
Kulturmedium:
    1% Glucose
    2% Melasse
    0,5% Sojamehl
    0,5% Caseinpepton
    0,2% $CaCl_2 \times 2H_2O$

pH-Wert: 6,3
Temperatur: 33°C
Rührerdrehzahl: 300 Upm
Luftzufuhr: 3 1/min

Nach 4 Tagen Fermentationsdauer war die Saccharosekonzentration (aus der Melasse) auf etwa Null abgesunken. Die Zellen wurden durch Zentrifugation abgetrennt und aus dem Kulturüberstand das bakterienlysierende Emzymprodukt durch Alkoholfällung, Ionen-austauscherchromatographie, Ultrafiltration und Gelfiltration isoliert.

In der folgenden Tabelle 3 wurde die im Kulturüberstand gefundene Aktivität an bakterienlysierendem Enzymprodukt gleich 100% Ausbeute gesetzte. Die in der Spalte "Ausbeute" angegebenen Prozentzahlen beziehen sich somit auf denjenigen Anteil, der mit dem in der ersten Spalte aufgeführten Isolierverfahren wiedergewonnen wird.

In der Spalte "Verfahren" bedeuten:
0 = keine Isolierung (Aktivität des Kulturüberstands)
1 = Ethanolfällung und Auflösung des Niederschlags in Puffer
2 = Kationenaustauscherchromatographie
3 = Ultrafiltration
4 = Gelfiltration

3

Aktivitätsbestimmung von bakterienlysierendem Enzymprodukt: Zu 2,8 ml einer Suspension von 0,2 mg Micrococcus luteus ATCC 4698 (Boeringer Mannheim) pro ml 0,1 M Natriumacetatpuffer (pH 5,0) werden 0,2 ml bakterienlysierendes Enzymprodukt-haltige Proben pipettiert und bei 25°C die Abnahme der Trübung durch Messung der Extinktion bei 450 nm bestimmt. Als 1 U wird die Extinktionsabnahme von 0,001 pro Minute definiert.

Proteinbestimmung: Methode nach Lowry, Rosenbrough, Farr und Randall, J. Biol. Chem. *193*, 265 (1951).

Beispiel 4: Lysewirkung

Die in der Tabelle 4 genannten Mikroorganismen werden 24 Stunden kultiviert, die Zellen abzentrifugiert (5 Minuten bei 1500 g), 2 × mit 0,1 N Natriumacetatpuffer (pH 5,0) gewaschen und in diesem Acetatpuffer gut suspendiert. In dieser Suspension wird die vorstehend genannte Aktivitätsbestimmung von bakterienlysierendem Enzymprodukt durchgeführt. In Tabelle 4a sind gram-positive, in Tabelle 4b gram-negative Bakterien aufgeführt. Als Vergleich dient jeweils Ei-Lysozym.

Tabelle 3: Isolierung des bakterienlysierenden Enzymprodukts

| Verfahren | eingesetzte Menge des Kulturüberstands [ml] | Enzymaktivität | | Proteinmenge im Kulturüberstand [mg/ml] | spezifische Aktivität [U/mg Protein] | Ausbeute [%] |
|---|---|---|---|---|---|---|
| | | [U/ml] | U/eingesetzter Menge $\cdot 10^6$ | | | |
| 0 | 4.400 | 10.800 | 47,5 | 5,84 | 1.850 | 100 |
| 1 | 120 | 376.100 | 45,1 | 134,3 | 2.800 | 94,9 |
| 2 | 210 | 200.470 | 42,1 | 14,4 | 13.922 | 88,6 |
| 3 | 60 | 685.000 | 41,1 | 43,3 | 15.820 | 86,5 |
| 4 | 180 | 212.300 | 38,2 | 10,2 | 20.814 | 80,4 |

EP 0 181 562 B1

EP 0 181 562 B1

### Tabelle 4a

| Sammlung-Nr. | Mikroorganismen | DSM 3030-bakterien-lysierendes Enzymprodukt %-Lyse nach: | | E1-Lysozym %-Lyse nach | |
|---|---|---|---|---|---|
| | | 30 min | 15 h | 30 min | 15 h |
| ATCC 10240 | Micrococcus flavus | 6,3 | 64,0 | 42,1 | 79,2 |
| | Micrococcus pyogenes | 29,8 | 53,0 | 11,4 | 41,5 |
| ATCC 6538 | Staphylococcus aureus | 0 | 21,7 | 2,1 | 18,3 |
| ATCC 10541 | Streptococcus faecalis | 8,8 | 44,2 | 0,5 | 11,9 |
| DSM 20200 | Leuconostoc cremoris | 16,1 | 73,1 | 1,8 | 11,8 |
| DSM 20193· | Leuconostoc mesenteroides | 16,6 | 46,0 | 0 | 7,7 |
| ATCC 9341 | Sarcina lutea | 0 | 17,5 | 1,2 | 15,0 |
| ATCC 9341 a | Sarcina lutea | 1,2 | 11,0 | 2,0 | 18,8 |
| ATCC 11778 | Bacillus cereus | 4,8 | 38,8 | 4,9 | 23,5 |

EP 0 181 562 B1

Fortsetzung Tabelle 4a

| Sammlung-Nr. | Mikroorganismen | DSM 3030-bakterien-lysierendes Enzymprodukt | | E1-Lysozym | |
|---|---|---|---|---|---|
| | | %-Lyse nach: | | %-Lyse nach | |
| | | 30 min | 15 h | 30 min | 15 h |
| ATCC 13732 | Clostridium butyricum | 0 | 34,3 | 0 | 0 |
| | Clostridium tyrobutyricum | 2,8 | 25,5 | 0 | 0 |
| | Clostridium pectinovorum | 21,2 | 69,4 | 8,2 | 27,0 |
| | Clostridium acetobutylicum | 0 | 47,3 | 0 | 0 |
| ATCC 11443 | Lactobacillus casei | 1,6 | 17,1 | 0 | 6,6 |
| ATCC 4963 | Lactobacillus acidophilus | 4,9 | 38,3 | 0 | 4,8 |
| DSM 2129 | Lactobacillus bulgaricus | 3,6 | 54,0 | 5,9 | 28,6 |
| ATCC 6946 | Corynebacterium simplex | 8,6 | 70,9 | 4,4 | 28,1 |
| ATCC 7005 | Corynebacterium hoagii | 4,6 | 46,2 | 4,0 | 34,7 |
| NCJB 9114 | Streptomyces aureofaciens | 33,8 | 55,4 | 18,7 | 41,4 |
| NRRL 3585 | Streptomyces clavuligerus | 44,4 | 90,1 | 12,9 | 35,7 |
| NRRL 8057 | Streptomyces cattleya | 41,9 | 65,2 | 19,8 | 57,6 |
| ATCC 21317 | Arthrobacter paraffineus | 10,3 | 25,9 | 2,9 | 19,3 |
| | Mycobacterium 607 | 12,0 | 27,5 | 18,9 | 27,0 |

EP 0 181 562 B1

Tabelle 4b

| Sammlung-Nr. | Mikroorganismen | DSM 3030-bakterien-lysierendes Enzymprodukt %-Lyse nach: | | E1-Lysozym %-Lyse nach | |
|---|---|---|---|---|---|
| | | 30 min | 15 h | 30 min | 15 h |
| ATCC 14909 | Pseudomonas alcaligenes | 39,6 | 56,9 | 15,4 | 33,0 |
| ATCC 13985 | Pseudomonas aureofaciens | 7,0 | 18,5 | 0 | 0 |
| ATCC 13525 | Pseudomonas fluorescens | 5,9 | 9,8 | 0 | 1,7 |
| ATCC 11172 | Pseudomonas fluorescens | 3,6 | 55,8 | 0 | 20,8 |
| ATCC 4973 | Pseudomonas fragi | 3,3 | 18,4 | 0 | 0 |
| ATCC 12099 | Pseudomonas rubescens | 5,3 | 13,1 | 0 | 0 |
| | Xanthomonas spez. | 3,4 | 8,3 | 2,2 | 5,1 |
| ATCC 11105 | Escherichia coli | 2,4 | 8,1 | 0,6 | 3,7 |
| | Escherichia coli | 45,6 | 61,9 | 14,0 | 22,2 |
| | Escherichia coli | 44,4 | 86,0 | 7,8 | 21,1 |
| | Escherichia coli | 50,2 | 77,3 | 17,0 | 23,5 |
| | Klebsiella pneumoniae | 43,9 | 72,5 | 28,7 | 47,4 |
| | Klebsiella pneumoniae | 34,2 | 16,7 | 1,1 | 2,1 |
| | Enterobacter spez. | 6,2 | 37,9 | 0,5 | 30,3 |
| | Aerobacter vinelandii | 17,5 | 42,9 | 8,0 | 7,6 |
| | Serratia marcescens | 5,3 | 15,2 | 0,6 | 2,3 |
| NRRL-B-3874 | Flavobacterium spez. | 3,5 | 33,6 | 0 | 11,5 |
| NRRL-B-5641 | Flavobacterium spez. | 2,4 | 27,5 | 0,8 | 10,9 |
| | Flavobacterium dehydrogen. | 18,2 | 37,3 | 2,9 | 7,6 |
| ATCC 31062 | Flavobacterium spez. | 2,5 | 23,9 | 2,4 | 15,8 |

8

Beispiel 5: Lysewirkung in Milch

Als praxisnahes Testsystem wurden 1 ml 24 Stunden alte Kulturen der in der Tabelle 5 genannten Bakterien mit 9 ml pasteurisierter Vollmilch und 1 ml Lösung mit 400 U bakterienlysierendem Enzymprodukt versetzt und sofort, nach 1 Stunde und nach 20 Stunden Proben entnommen und darin die Lebendkeimzahl bestimmt. Als Vergleichswert wird in dem Testsystem anstelle der Lösung mit bakterienlysierendem Enzymprodukt 1 ml steriles Wasser eingesetzt.

Vergleichsbeispiel

Der Stamm DSM 3030 wurde mit dem in der Deutschen Offenlegungsschrift 21 46 597 genannten S. globisporium ATCC 21553 hinsichtlich der Ausbeute an bakterienlysierendem Enzymprodukt verglichen. In der Tabelle 6 bedeutet "Medium A" das im Beispiel 2 definierte, "Medium B" das im "Bezugsbeispiel 1" der Deutschen Offenlegungsschrift 21 46 597 genannte flüssige Medium:

Tabelle 6

| Medium | Stamm | Enzymaktivität im Kulturüberstand nach ... Tagen in U/ml | | | | | | |
|--------|-------|------|------|------|------|------|------|------|
| | | 2 | 3 | 4 | 5 | 6 | 7 | 8 |
| A | DSM 3030 | 253 | 788 | 2036 | 3478 | 4130 | 5605 | 6600 |
| | ATCC 21553 | 0 | 0 | 98 | 308 | 596 | 683 | 643 |
| B | DSM 3030 | 0 | 0 | 290 | 1250 | 1664 | 2068 | 2280 |
| | ATCC 21553 | 0 | 0 | 276 | 859 | 1130 | 1171 | 1247 |

EP 0 181 562 B1

Tabelle 5

| Gram | Bakterien | Keimzahl-bestimmung nach | Kein Lysozym (Vergleichswert) Keime / mlx $10^5$ | DSM 3030-bakterien-lysierendes Enzym-produkt Keime/mlx $10^5$ | Ei-Lysozym Keime/mlx $10^5$ |
|---|---|---|---|---|---|
| + | Corynebacterium simplex | 1 h | 7 700 | 3 900 | 2 400 |
| | ATCC 6946 | 20 h | 33 000 | 3,5 | 120 |
| + | Bacillus subtilis | 1 h | 560 | 450 | 550 |
| | | 20 h | 950 | 530 | 840 |
| + | Lactobacillus casei | 1 h | 200 | 210 | 290 |
| | ATCC 11443 | 20 h | 1 300 | 1 500 | 2 000 |
| + | Clostridium pectino-vorum | 1 h | 460 | 210 | 580 |
| | | 20 h | 1 200 | 140 | 300 |
| – | Escherichia coli | 1 h | 5,1 | 42 | 38 |
| | | 20 h | 340 | 20 | 23 |
| – | Escherichia coli | 1 h | 36 | 28 | 40 |
| | | 20 h | 2 400 | 16 | 82 |
| – | Klebsiella pneumoniae | 1 h | 700 | 580 | 770 |
| | | 20 h | 1 100 | 550 | 130 |
| – | Serratia marcescens | 1 h | 360 | 50 | 55 |
| | | 20 h | – | – | – |

# EP 0 181 562 B1

**Patentansprüche für die Vertragsstaaten: BE, CH DE, FR, GB, IT, LI, LU, NL, SE**

1. Bakterienlysierendes Enzymprodukt mit einem Aktivitätsbereich von ca. pH 3 bis ca. pH 9, einem Temperaturoptimum von 50 bis 60°C und einem pH-Optimum von 4, 5 bis 5, erhältlich durch Fermentation von Streptomyces coelicolor DSM 3030.

2. Verfahren zur Herstellung eines bakterienlysierenden Enzymprodukts, gemäß Anspruch 1, durch Fermentation von Streptomyceten, dadurch gekennzeichnet, daß man den Stamm Streptomyces coelicolor DSM 3030 einsetzt.

3. Verfahren nach Anspruch 2, dadurch gekennzeichnet, daß das Fermentationsmedium Zuckerrübenmelasse enthält.

4. Verfahren nach Anspruch 2 oder 3, dadurch gekennzeichnet, daß das Fermentationsmediuim Calciumionen enthält.

5. Verwendung des bakterienlysierenden Enzymprodukts gemäß Anspruch 1 bzw. des nach Anspruch 2 bis 4 erhaltenen bakterienlysierenden Enzymprodukts zur Konservierung von Lebensmitteln, Verhinderung von Infektionen und Protoplastenherstellung.

6. Streptomyces coelicolor DSM 3030 und seine Mutanten und Varianten, sofern sie ein bakterienlysierendes Enzymprodukt, gemäß Anspruch 1, bilden.

**Patentansprüche für den Vertragsstaat: AT**

1. Verfahren zur Herstellung eines bakterienlysierenden Enzymprodukts, mit einem Aktivitätsbereich von ca. pH 3 bis ca. pH 9, einem Temperaturoptimum von 50 bis 60°C und einem pH-Optimum von 4,5 bis 5,0, durch Fermentation von Streptomyceten, dadurch gekennzeichnet, daß man den Stamm Streptomyces coelicolor DSM 3030 einsetzt.

2. Verfahren nach Anspruch 1, dadurch gekennzeichnet, daß das Fermentationsmedium Zuckerrübenmelasse enthält.

3. Verfahren nach Anspruch 1 oder 2, dadurch gekennzeichnet, daß das Fermentationsmedium Calciumionen enthält.

4. Verwendung des bakterienlysierenden Enzymprodukts, erhältlich nach Anspruch 1 zur Konservierung von Lebensmitteln, Verhinderung von Infektionen und Protoplastenherstellung.

5. Streptomyces coelicolor DSM 3030 und seine Mutanten und Varianten, sofern sie ein bakterienlysierendes Enzymprodukt, gemäß Anspruch 1, bilden.

**Revendications pour les Etats Contractants: BE, CH, DE, FR, GB, IT, LU, NL, SE**

1. Produit enzymatique bactériolytique ayant un domaine d'activité allant d'environ pH 3 à environ pH 9, un optimum de températures de 50 à 60°C et un optimum de pH de 4,5 à 5, pouvant être obtenu par fermentation au moyen de *Streptomyces coelicolor* DSM 3030.

2. Procédé pour la protection, selon la revendication 1, d'un produit enzymatique bactériolytique par fermentation au moyens de *Streptomyces,* caractérisé en ce que l'on utilise la souche *Streptomyces coelicolor* DSM 3030.

3. Procédé selon la revendication 2, caractérisé en ce que le milieu de fermentation contient de la mélasse de betterave à sucre.

4. Procédé selon la revendication 2 ou 3, caractérisé en ce que le milieu de fermentation contient des ions calcium.

5. Utilisation de produit enzymatique bactériolytique selon la revendication 1 ou du produit enzymatique bactériolytique obtenu selon les revendications 2 à 4, pour la conservation de produits alimentaires, l'empêchement d'infections et la production de protoplastes.

6. *Streptomyces coelicolor* DSM 3030 et ses mutants et variants, dans la mesure où ils élaborent un produit enzymatique bactériolytique selon la revendication 1.

**Revendications pour l'Etat Contractant: AT**

1. Procédé pour la production d'un produit enzymatique bactériolytique ayant un domaine d'activité d'environ pH 3 à environ pH 9, un optimum de températures de 50 à 60°C et un optimum de pH de 4,5 à 5,0, par fermentation au moyen de *Streptomyces,* caractérisé en ce que l'on utilise la souche *Streptomyces coelicolor* DSM 3030.

2. Procédé selon la revendication 1, caractérisé en ce que le milieu de fermentation contient de la mélasse de betterave à sucre.

3. Procédé selon la revendication 1 ou 2, caractérisé en ce que le milieu de fermentation contient des ions calcium.

4. Utilisation de produit enzymatique bactériolytique, pouvant être obtenu selon la revendication 1, pour la conservation de produits alimentaires, l'empêchement d'infections et la production de protoplastes.

11

# EP 0 181 562 B1

5. *Streptomyces coelicolor* DSM 3030 et ses mutants et variants, dans la mesure où ils élaborent, selon la revendication 1, un produit enzymatique bactériolytique.

## Claims for the Contracting States: BE, CH, DE, FR, GB, IT, LI, LU, NL, SE

1. A Baterialytic enzyme product having an activity range of approximately pH 3 to approximately pH 9, a temperature optimum from 50 to 60°C and a pH optimum from 4.5 to 5 and obtainable by fermentation of Streptomyces coelicolor DSM 3030.

2. A process for the preparation of a bacteriolytic enzyme product as claimed in claim 1 by fermentation of Streptomycetes, which comprises the use of the strain Streptomyces coelicolor DSM 3030.

3. The process as claimed in claim 2, wherein the fermentation medium contains sugar beet molasses.

4. The process as claimed in claim 2 or 3, wherein the fermentation medium contains calcium ions.

5. The use of the bacteriolytic enzyme product as claimed in claim 1, or of the bacteriolytic enzyme product obtained as claimed in claim 2 to 4, for the preservation of foodstuffs, the prevention of infections and the preparation of protoplasts.

6. Streptomyces coelicolor DSM 3030, and those of its mutants and variants which form a bacteriolytic enzyme product as claimed in claim 1.

## Claims for the Contracting State: AT

1. A process for the preparation of a bacteriolytic enzyme product having an activity range from approximately pH 3 to approximately pH 9, a temperature optimum from 50 to 60°C and a pH optimum from 4.5 to 5.0, by fermentation of Streptomycetes, which comprises the use of the strain Streptomyces coelicolor DSM 3030.

2. The process as claimed in claim 1, wherein the fermentation medium contains sugar beet molasses.

3. The process as claimed in claim 1 or 2, wherein the fermentation medium contains calcium ions.

4. The use of the bacteriolytic enzyme product obtained as claimed in claim 1 for the preservation of foodstuffs, the prevention of infections and the preparation of protoplasts.

5. Streptomyces coelicolor DSM 3030, and those of its mutants and variants which form a bacteriolytic enzyme product as claimed in claim 1.